(19) Europäisches Patentamt
European Patent Office
Office européen des brevets

(11) Publication number: 0 072 055 B1

(12) # EUROPEAN PATENT SPECIFICATION

(45) Date of publication of patent specification: 02.05.85

(21) Application number: 82200864.5

(22) Date of filing: 09.07.82

(51) Int. Cl.$^4$: **C 07 C 51/12**, C 07 C 53/08, C 07 C 53/122, C 07 C 53/124, C 07 C 63/06, C 07 C 53/02, C 07 C 67/36, C 07 C 67/37, C 07 C 69/14, C 07 C 69/24, C 07 C 69/16

(54) **Process for the co-production of carboxylic acids and carboxylic acid esters.**

(30) Priority: 06.08.81 GB 8124111

(43) Date of publication of application: 16.02.83 Bulletin 83/07

(45) Publication of the grant of the patent: 02.05.85 Bulletin 85/18

(84) Designated Contracting States: BE DE FR GB IT NL

(56) References cited:
EP-A-0 031 606
EP-A-0 046 128
EP-A-0 046 129
WO-A-81/00856
GB-A-1 450 993

(73) Proprietor: SHELL INTERNATIONALE RESEARCH MAATSCHAPPIJ B.V.
Carel van Bylandtlaan 30
NL-2596 HR Den Haag (NL)

(72) Inventor: Drent, Eit
Badhuisweg 3
NL-1031 CM Amsterdam (NL)

(74) Representative: Aalbers, Onno et al
P.O. Box 302
NL-2501 CH The Hague (NL)

EP 0 072 055 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to a process for the co-production of carboxylic acids and carboxylic acid esters from carboxylic acid esters having one carbon atom less in the molecule, carbon monoxide and hydrogen in the presence of a catalytic system. The invention relates in particular to a process for the co-production of acetic acid and ethyl acetate from methyl acetate under mild process conditions. Carboxylic acid esters produced according to the process according to the present invention are thus homologues of the carboxylic acid esters used as starting materials.

The production of carboxylic acid esters via homologation has already been described in the literature. For instance, it is known from Dutch published patent application 7807520 that carboxylic acid esters (especially ethyl acetate from methyl acetate) can be prepared at elevated temperatures at a pressure of at least 100 bar, preferably between 200 and 1500 bar in the presence of cobalt, rhodium, ruthenium, or iron or salts thereof. Apart from the fact that very high pressures have to be applied in order to get a reasonable conversion, the process as described also has the disadvantage that water is produced as the co-product. It will be clear that water can cause hydrolysis of the esters present in the reaction mixture (whether being present as product or as starting material). Even when very high, unattractive pressures (e.g. well over 1000 bar) are used substantial amounts of alkanols are produced.

It is further known from German Offenlegungsschrift 2733663 that the homologation of methyl acetate (or of its precursor dimethyl ether) can be carried out using a ruthenium carbonyl compound and an iodide or bromide promoter at elevated temperatures and pressures. The process as described in the German Offenlegungsschrift also has to be carried out at very high pressures (well over 200 bar) and substantial amounts of other products (including not only alkanols but also methane) are formed.

It is known from Dutch published patent application 7602096 that methyl acetate can be converted using carbon monoxide and hydrogen in the presence of a specific catalyst comprising a Group VIII noble metal compound and a halogen (especially iodine) source into acetic acid and ethylidene diacetate. However, ethyl acetate (the homologation product according to the process according to the present invention) is not even mentioned as a by-product.

European Patent Application No. 31606 discloses that by the use of a very specific catalytic system, the stoichiometry of the known reactions of methyl acetate with carbon monoxide and hydrogen can be altered most advantageously to produce one mole of ethyl acetate and two moles of acetic acid from two moles of methyl acetate. However this system requires the use of a catalyst containing three metallic elements, including a Group II or transition metal iodide or bromide, especially zinc iodide, which can be rather expensive. In addition, problems may occur in the work-up procedure due to the presence of three different metals. In some cases, crystallisation of the Group II or transition metal iodide or bromide may be a problem.

A catalyst system has now been found whereby the desirable stoichiometry of the reaction of European Patent Application No. 31606 can be retained, the catalyst system requiring the presence of only two different metallic elements.

The present invention provides a process for the co-production of carboxylic acids of the general formula $R^1$—COOH and $R^2$—COOH and carboxylic acid esters of the general formula $R^1$—$COOCH_2R^2$ and $R^2$—$COOCH_2R^1$ wherein each of the groups $R^1$ and $R^2$, which may be the same or different, represents an alkyl group having from 1 to 20 carbon atoms which may be substituted by fluorine or chlorine or represent an aryl, alkaryl or aralkyl group which may be substituted by fluorine or chlorine whilst $R^1$ may also represent a hydrogen atom, characterized in that a carboxylic acid ester of the general formula $R^1$—$COOR^2$ and/or an ether of the general formula $R^3OR^4$, wherein $R^1$ and $R^2$ are as defined hereinbefore and each of $R^3$ and $R^4$, which may be the same or different, represents an alkyl group having from 1 to 20 carbon atoms which may be substituted by fluorine or chlorine or represent an aryl, alkaryl or aralkyl group which may be substituted by fluorine or chlorine, is reacted with carbon monoxide and hydrogen at elevated temperature and pressure in the presence of a catalytic system which comprises a ruthenium compound and a further Group VIII metal compound, hydrogen iodide, hydrogen bromide, elemental iodine and/or elemental bromine being added to the reaction mixture.

It should be noted that the composition of the reaction product mixture will be governed by the choice of the starting carboxylic acid esters and/or ethers. For instance, when starting materials are used wherein the groups $R^1$ and $R^2$ are identical, such as in methyl acetate, dimethyl ether and ethyl propionate, the reaction product mixture will normally contain only the carboxylic acid ester homologue and the appropriate acid. When starting materials are used wherein the groups $R^1$ and $R^2$ are not identical, a more complex reaction product mixture will be obtained which comprises normally at least two carboxylic acid ester homologues and two appropriate carboxylic acids. For instance, when ethyl acetate is used as the starting material the reaction product mixture comprises propyl acetate, ethyl propionate, propionic acid and acetic acid.

It will be appreciated that any carboxylic acid ester homologue produced according to the present process can serve as starting material in the process according to the present invention thus forming the next carboxylic acid ester homologue(s) and the appropriate carboxylic acid(s). In addition, since carboxylic acids are produced in

the process according to the present invention, transesterification reactions, i.e. reactions between carboxylic acids and carboxylic acid esters, or between different carboxylic acid esters, may also occur under the prevailing reaction conditions. It will be clear that transesterification reactions do not alter the product composition when the starting material comprises compounds wherein $R^1$ and $R^2$ are identical, but may alter the product composition when the groups $R^1$ and $R^2$ are not identical.

For the purpose of the present invention, carboxylic acids and carboxylic acid esters, obtained via a further homologation of produced carboxylic acid ester, or obtained by a transesterification process under the prevailing conditions, are considered to be within the scope of the present invention.

From the above it will be clear that preference is given to processes wherein starting materials are used wherein the groups $R^1$ and $R^2$ are identical since a less complex reaction mixture will be obtained. The process according to the present invention is of special interest for the co-production of acetic acid and ethyl acetate from methyl acetate according to the equation:

$$2CH_3COOCH_3+2CO+2H_2 \rightarrow CH_3COOC_2H_5+2CH_3COOH$$

since the products can be obtained with high selectivity and close to the stoichiometrically expected ratio. This is of special interest when the process according to the invention is part of an integrated process, wherein acid produced—for instance acetic acid—is to be recycled in the process. Moreover, the process according to the present invention can be carried out conveniently at surprisingly low pressures, e.g. pressures well below 100 bar can be used advantageously.

Suitable starting materials which can be used conveniently in the process according to the present invention include compounds of the general formula $R^1$—$COOR^2$ and/or $R^3OR^4$, wherein each of $R^1$, $R^2$, $R^3$ and $R^4$, which may be the same or different, represents an alkyl group having from 1 to 12 carbon atoms, or an aryl, alkaryl or aralkyl group having up to 12 carbon atoms, whilst $R^1$ may also represent a hydrogen atom. Preference is given to the use of compounds of the general formula $R^1$—$COOR^2$ and/or $R^3OR^4$, wherein $R^1$, $R^2$, $R^3$ and $R^4$ are the same and each represents an alkyl group having from 1 to 12 carbon atoms or an aryl, alkaryl or aralkyl group having up to 12 carbon atoms. Most preferred starting materials are methyl acetate and dimethyl ether.

When ethers of the general formula $R^3OR^4$ are used as starting materials in the process according to the present invention, it would appear that these compounds will be converted primarily into the corresponding esters by the introduction of a carbon monoxide moiety into the molecule which molecule may then undergo the homologation reaction according to the present invention. If desired, the reaction according to the present invention may be carried out in two stages when an ether is used as the starting material. Firstly, the ether is converted into the corresponding ester which in its turn, in the same or in a different vessel, is converted into the final products. If desired, mixtures of carboxylic acid esters and/or ethers can be used as starting materials.

Ruthenium compounds which can be used conveniently in the process according to the present invention include ruthenium (III) chloride, ruthenium (III) chloride trihydrate, ruthenium (IV) chloride, ruthenium (III) bromide, the ruthenium oxides, organic ruthenium salts such as ruthenium (III) propionate, ruthenium (III) butyrate, ruthenium pentacarbonyl, triruthenium-dodecacarbonyl and mixed ruthenium halocarbonyls such as bis-(rutheniumtricarbonyl-dibromide), and other organoruthenium complexes.

Further Group VIII metal compounds which can be used together with a ruthenium compound in the catalytic system include palladium and, especially, rhodium compounds, although other Group VIII metal compounds can also be used. Examples of suitable rhodium compounds include rhodium oxide, rhodium (III) hydroxide, rhodium (III) chloride, rhodium (III) chloride trihydrate, rhodium (III) bromide, rhodium (III) iodide and the corresponding pyridine and phosphine complexes such as tris(pyridine) rhodium (III) chloride or dichloro bis-(triphenylphosphine) rhodium, rhodium (III) formate, rhodium (III) acetate, rhodium (III) butyrate, rhodium (III) naphthenate, dirhodium octacarbonyl, tetrarhodium dodecacarbonyl, hexarhodium hexadecacarbonyl, rhodium dicarbonylacetylacetonate and other organo-rhodium complexes. Preference is given to the use of rhodium (III) chloride trihydrate.

Examples of suitable palladium compounds include palladium chloride, palladium chloride dihydrate, palladium bromide, palladium iodide, palladium oxide, or an organic palladium salt or complex such as palladium formate, palladium acetate, palladium butyrate and palladium acetylacetonate. Preferred palladium compounds are palladium chloride, palladium chloride dihydrate and palladium acetate.

The molar ratio of ruthenium compound to further Group VIII metal compound is not critical and can vary between wide limits, e.g. atomic ratios of ruthenium to further Group VIII metal between 50:1 and 1:20, especially 10:1 and 1:5, are suitable.

The amount of ruthenium compound and further Group VIII metal compound to be used is not critical and any amount which exerts catalytic activity can be used. Amounts as low as 0.001 %w, calculated on carboxylic acid ester or ether to be converted can be used, preference being given to amounts in the range of from 0.01—10 %w, most preferably between 0.05—5 %w.

The quantity of hydrogen iodide, hydrogen

bromide, elemental iodine and/or elemental bromine added is not crucial. Suitably the atomic ratio of added iodine plus bromine to total Group VIII metal is in the range of from 0.1:1 to 200:1, preferably 1:1 to 100:1, and especially 10:1 to 50:1. Hydrogen iodide is a preferred additive for the reaction mixture.

If desired, the process according to the present invention can be carried out in the additional presence of organic promotors of the type commonly used in catalytic processes. Suitable promotors include compounds which are capable of forming a coordination compound with the Group VIII metal moieties present in the catalytic system according to the present invention. Suitable promotors of this type include organo-phosphorus, organo-arsenic, organic-antimony, organo-nitrogen, organo-sulphur and organo-oxygen compounds having a lone pair of electrons. Preferred compounds of this type are organo-phosphorus compounds and organo-nitrogen compounds. Compounds containing both phosphorus and oxygen atoms or nitrogen and oxygen atoms can be used.

Examples of organo-nitrogen compounds which can be used conveniently include amines such as pyrrole, alkyl-substituted pyrroles, pyrrolidine, alkyl-substituted pyrrolidines, pyridine, alkyl-substituted pyridines, piperidines, alkyl-substituted piperidines, pyrimidine, alkyl-substituted pyrimidines, pyrazine, benztriazole, tetraethylenediamine, 1,10-phenanthroline, alkyl-substituted 1,10-phenanthrolines, morpholine and alkyl-substituted morpholines. Preference is given to the use of pyridine and alkyl-substituted pyridines such as the various picolines, e.g. alpha-picoline.

Examples of organo-phosphorus compounds which can be used conveniently include tertiary phosphines of the general formula $PR^5R^6R^7$, wherein each of $R^5$, $R^6$ and $R^7$, which may be the same or different, represents an alkyl, cycloalkyl or aryl group having up to 10 carbon atoms. Preferred organo-phosphorus compounds comprise trimethylphosphine, triethylphosphine, tri-n-butylphosphine and triphenylphosphine. Also the corresponding organo-phosphites of the general formula $P.OR^5.OR^6.OR^7$ (wherein $R^5$, $R^6$ and $R^7$ are as defined hereinbefore) can be used as coordination compounds.

There may also be present in the reaction mixture as promotor one or more phosphorus V compounds which do not possess a lone pair of electrons. Such compounds include phosphine oxides, e.g. $OP.R^5R^6R^7$, and esters of various phosphorus acids, e.g. $OP.OR^5.(O)mR^6.(O)mR^7$, where $R^5$, $R^6$ and $R^7$ have the meanings given above, and each m independently is 0 or 1.

The optimum amount of organic promotor to be used in the process according to the present invention will of course vary depending upon the precise catalytic system used. In general, amounts of from 0.01—100 moles of promotor per gram atom of ruthenium can be suitably applied. Preference is given to the use of an amine or a phosphine as promotor in an amount of less than 0.5, especially less than 0.3, moles of amine or phosphine per gram atom of ruthenium. It should be stated, however, that the presence of promotors is optional.

The process according to the present invention can be carried out using a wide range of temperatures. Temperatures up to 300°C can be suitably applied. Preference is given to temperatures in the range of from 50°C to 200°C, most preferred temperatures are in the range between 125°C and 175°C.

The process according to the present invention can be carried out using low pressures, e.g. pressures as low as 5 bar. Pressures in the range of from 20 to 100 bar are preferred. Higher pressures, e.g. pressures as high as 1000 bar can be applied, but they are generally not economical because of the investment and energy costs involved.

According to the reaction equation carbon monoxide and hydrogen are consumed in a molar ratio of 1:1. It has been found, however, that without any substantial disadvantage wider molar ratios, e.g. ratios of from 1:10 to 10:1 can be applied. Preference is given to ratios carbon monoxide: hydrogen in the range of from 1:0.5 to 1:2.

The process according to the present invention may be carried out in the presence of a solvent. Suitable solvents include carboxylic acids such as acetic acid or propanoic acid; carboxylic acid esters, such as methyl acetate, ethyl acetate, methylpropionate or ethyl propionate (being used as solvent as well as starting material), and cyclic ethers such as tetrahydrofuran, 1,4-dioxane, 1,3-dioxane and the dioxolanes. Also dialkyl ethers used in excess as starting material may be regarded as solvent for the process according to the present invention. Suitable dialkylethers include dimethyl ether, diethyl ether and methyl t-butyl ether.

Other compounds which can be used as solvent in the process according to the present invention include sulphones and sulphoxides. Examples of such compounds are dimethylsulphone, sulpholane, 2-methyl sulpholane, 3-methyl sulpholane, dimethylsulphoxide and diethyl sulphoxide.

It has been found that the mild conditions according to the present invention even tolerate the presence of some water in the reaction medium. Although the presence of water is not preferred, amounts of up to 15 %w, based on total solvent, can be present.

The process according to the present invention can be carried out in the liquid phase or in the gaseous phase. Preference is given to a liquid phase which enables a convenient introduction of carbon monoxide and hydrogen into the reaction vessel. If desired, the carbon monoxide and hydrogen can be introduced together into the reaction vessel. The process according to the present invention can be carried out batchwise, semicontinuously or continuously.

The process according to the present invention

is also of interest in that it can be integrated with known processes, either for the production of the starting materials (i.e. carboxylic acid esters or the corresponding ethers) or for the conversion of the carboxylic acid esters produced into other products, e.g. by transesterification processes. For instance, when the present process produces ethyl acetate, it can be integrated with a process for the preparation of methyl acetate from acetic acid and methanol using an acidic catalyst. Since the present process produces acetic acid, that compound may be recycled to serve as feedstock for the preparation of methyl acetate. If desired, the present process can also be integrated with a transesterification process, wherein ethyl acetate is transesterified with methanol to give methyl acetate (which can be recycled to serve as feedstock for the present process) and ethanol which can either be sold as such or converted into other products such as ethylene. In such a case acetic acid and/or methyl acetate can be removed from the system in an amount equimolar with ethanol produced.

The following Examples illustrate the invention.

Example

The experiment was carried out in a 300 ml magnet-driven autoclave of Hastealloy C (Trade Mark) which contained 25 ml methyl acetate, 25 ml acetic acid, 0.5 mmol rhodium (III) chloride trihydrate and 1 mmol ruthenium (III) chloride trihydrate. The vessel was flushed with carbon monoxide, and 50 mmol gaseous hydrogen iodide were introduced. The autoclave was then pressurised with carbon monoxide (20 bar partial pressure) and hydrogen (20 bar partial pressure). The autoclave was then heated to 160°C and kept at this temperature for 5 hours, during which time the pressure decreased as carbon monoxide and hydrogen were consumed. After this time the reaction mixture was analysed by gas-liquid chromatography and shown to contain 10.8 %w ethyl acetate. On a molar basis the conversion of the starting material was about 40% with an almost 100% selectivity towards the two products ethyl acetate and acetic acid. Only traces (less than 0.5%) of by-products were detected: in particular, no alcohols were detected.

**Claims**

1. A process for the co-production of carboxylic acids of the general formula $R^1$—COOH and $R^2$—COOH and carboxylic acid esters of the general formula $R^1$—$COOCH_2R^2$ and $R^2$—$COOCH_2R^1$, wherein each of the groups $R^1$ and $R^2$, which may be the same or different, represents an alkyl group having from 1 to 20 carbon atoms which may be substituted by fluorine or chlorine or represent an aryl, alkaryl or aralkyl group which may be substituted by fluorine or chlorine whilst $R^1$ may also represent a hydrogen atom, characterized in that a carboxylic acid ester of the general formula $R^1$—$COOR^2$ and/or an ether of the general formula $R^3OR^4$ wherein $R^1$ and $R^2$ are as defined hereinbefore and each of $R^3$ and $R^4$, which may be the same or different, represents an alkyl group having from 1 to 20 carbon atoms which may be substituted by fluorine or chlorine or represent an aryl, alkaryl or aralkyl group which may be substituted by fluorine or chlorine, is reacted with carbon monoxide and hydrogen at elevated temperature and pressure in the presence of a catalytic system which comprises a ruthenium compound and a further Group VIII metal compound, hydrogen iodide, hydrogen bromide, elemental iodine and/or elemental bromine being added to the reaction mixture.

2. A process according to claim 1 characterized in that as starting materials are used compounds of the general formula $R^1$—$COOR^2$ and/or $R^3OR^4$ wherein each of $R^1$, $R^2$, $R^3$ and $R^4$ which may be the same or different, represents an alkyl group having from 1 to 12 carbon atoms or an aryl, alkaryl or aralkyl group having up to 12 carbon atoms, whilst $R^1$ may also represent a hydrogen atom, and preferably are the same and represent an alkyl group having from 1 to 12 carbon atoms or an aryl, alkaryl or aralkyl group having up to 12 carbon atoms.

3. A process according to claim 1 or 2 characterized in that as ruthenium compound is used ruthenium (III) chloride, ruthenium (III) chloride trihydrate, ruthenium (IV) chloride, ruthenium (III) bromide, ruthenium oxide or an organic ruthenium salt or complex.

4. A process according to any one of the preceding claims characterized in that as further Group VIII metal compound is used a rhodium compound such as rhodium oxide, rhodium (III) hydroxide, rhodium (III) chloride, rhodium (III) chloride trihydrate, rhodium (III) bromide, rhodium (III) iodide or an organic rhodium salt or complex, preferably rhodium (III) chloride trihydrate, or a palladium compound such as palladium chloride, palladium chloride dihydrate, palladium bromide, palladium iodide, palladium oxide or an organic palladium salt or complex, preferably palladium chloride, palladium chloride dihydrate or palladium acetate.

5. A process according to claim 4, characterized in that a rhodium compound is used as further Group VIII metal compound.

6. A process according to any one of the preceding claims, characterized in that the ruthenium compound and the further Group VIII metal compound are used in a ratio between 50:1 and 1:20, preferably between 10:1 and 1:5.

7. A process according to any one of the preceding claims characterized in that additionally an organo-phosphorus, organo-arsenic, organo-antimony, organo-nitrogen, organo-sulphur or organo-oxygen compound is present in the reaction mixture as a promotor.

8. A process according to claim 7, in which an amine or a phosphine is present in the reaction mixture in an amount less than 0.5 moles per gram atom of ruthenium.

9. A process according to any one of the preceding claims characterized in that the reaction is carried out at a temperature in the range of from 50°C to 200°C, and especially between 125°C and 175°C.

10. A process according to any one of the preceding claims characterized in that the process is carried out using a pressure between 20 and 100 bar.

**Patentansprüche**

1. Verfahren zur gleichzeitigen Herstellung von Carbonsäuren der allgemeinen Formel $R^1$—COOH und $R^2$—COOH und Carbonsäureestern der allgemeinen Formel $R^1$—$COOCH_2R^2$ und $R^2$—$COOCH_2R^1$, wobei jeder der Reste $R^1$ und $R^2$, die gleich oder verschieden sein können, eine Alkylgruppe mit 1 bis 20 Kohlenstoffatomen bedeutet, die durch Fluor oder Chlor substituiert sein kann, oder eine Aryl-, Alkaryl- oder Aralkylgruppe bedeutet, die durch Fluor oder Chlor substituiert sein kann, während $R^1$ auch ein Wasserstoffatom bedeuten kann, dadurch gekennzeichnet, daß ein Carbonsäureester der allgemeinen Formel $R^1$—$COOR^2$ und/oder ein Ether der allgemeinen Formel $R^3$—$OR^4$, wobei $R^1$ und $R^2$ wie oben definiert sind und $R^3$ und $R^4$ gleich oder verschieden sein können und jeweils eine Alkylgruppe mit 1 bis 20 Kohlenstoffatomen bedeuten, die durch Fluor oder Chlor substituiert sein kann, oder eine Aryl-, Alkaryl- oder Aralkylgruppe bedeuten, die durch Fluor oder Chlor substituiert sein kann, umgesetzt wird mit Kohlenmonoxid und Wasserstoff bei erhöhter Temperatur und Druck in Gegenwart eines katalytischen Systems, das eine Rutheniumverbindung und eine weitere Verbindung eines Metalls der Gruppe VIII enthält, wobei Jodwasserstoff, Bromwasserstoff, elementares Jod und/oder elementares Brom zu dem Reaktionsgemisch zugesetzt werden.

2. Verfahren nach Anspruch 1, dadurch gekennzeichnet, daß als Ausgangsmaterialien Verbindungen der allgemeinen Formel $R^1COOR^2$ und/oder $R^3OR^4$ angewandt werden, bei denen jeder der Reste $R^1$, $R^2$, $R^3$ und $R^4$, die gleich oder verschieden sein können, eine Alkylgruppe mit 1 bis 12 Kohlenstoffatomen oder eine Aryl-, Alkaryl- oder Aralkylgruppe mit bis zu 12 Kohlenstoffatomen bedeutet, während $R^1$ auch ein Wasserstoffatom bedeuten kann und (die Reste $R^1$, $R^2$, $R^3$ und $R^4$) vorzugsweise gleich sind und eine Alkylgruppe mit 1 bis 12 Kohlenstoffatomen oder eine Aryl-, Alkaryl- oder Aralkylgruppe mit bis zu 12 Kohlenstoffatomen bedeuten.

3. Verfahren nach Anspruch 1 oder 2, dadurch gekennzeichnet, daß als Rutheniumverbinding Ruthenium(III)-chlorid, Ruthenium(III)-chlorid-trihydrat, Ruthenium(IV)-chlorid, Ruthenium(III)-bromid, Rutheniumoxid oder ein organisches Rutheniumsalz oder ein Komplex verwendet wird.

4. Verfahren nach einem der vorangehenden Ansprüche, dadurch gekennzeichnet, daß als weitere Verbindung eines Metalls der Gruppe VIII eine Rhodiumverbindung verwendet wird wie Rhodiumoxid, Rhodium(III)-hydroxid, Rhodium(III)-chlorid, Rhodium (III)-chlorid-trihydrat, Rhodium(III)-bromid, Rhodium-(III)-jodid oder ein organisches Rhodiumsalz oder ein Komplex, vorzugsweise Rhodium(III)-chlorid-trihydrat oder eine Palladiumverbindung wie Palladiumchlorid, Palladiumchloriddihydrat, Palladiumbromid, Palladiumjodid, Palladiumoxid oder ein organisches Palladiumsalz oder ein Komplex, vorzugsweise Palladiumchlorid, Palladiumchlorid-dihydrat oder Palladiumacetat.

5. Verfahren nach Anspruch 4, dadurch gekennzeichnet, daß eine Rhodiumverbindung als weitere Verbindung eines Metalls der Gruppe VIII verwendet wird.

6. Verfahren nach einem der vorangehenden Ansprüche, dadurch gekennzeichnet, daß die Rutheniumverbindung und die weitere Verbindung eines Metalls der Gruppe VIII in einem Verhältnis zwischen 50:1 und 1:20 vorzugsweise zwischen 10:1 und 1:5 verwendet werden.

7. Verfahren nach einem der vorangehenden Ansprüche, dadurch gekennzeichnet, daß zusätzlich eine Organophosphor-, Organoarsen,-, Organoantimon-, Organostickstoff-, Organoschwefel- oder Organosauerstoff-Verbindung in dem Reaktionsgemisch als Promotor vorhanden ist.

8. Verfahren nach Anspruch 7, wobei ein Amin oder ein Phosphin in dem Reaktionsgemisch in einer Menge von weniger als 0,5 Mol/g-Atom Ruthenium vorhanden ist.

9. Verfahren nach einem der vorangehenden Ansprüche, dadurch gekennzeichnet, daß die Reaktion bei einer Temperatur im Bereich von 50°C bis 200°C und besonders zwischen 125°C und 175°C durchgeführt wird.

10. Verfahren nach einem der vorangehenden Ansprüche, dadurch gekennzeichnet, daß das Verfahren bei einem Druck zwischen 20 und 100 bar durchgeführt wird.

**Revendications**

1. Un procédé pour la coproduction d'acides carboxyliques des formules générales $R^1$—COOH et $R^2$—COOH et d'esters d'acides carboxyliques des formules générales $R^1$—$COOCH_2R^2$ et $R^2$—$COOCH_2R^1$, où les groupes $R^1$ et $R^2$, qui peuvent être identiques ou différents, représentent chacun un groupe alcoyle ayant de 1 à 20 atomes de carbone qui peut être substitué par du fluor ou du chlore ou représentent un groupe aryle, alcaryle ou aralcoyle qui peut être substitué par du fluor ou du chlore tandis que $R^1$ peut aussi représenter un atome d'hydrogène, caractérisé en ce qu'un ester d'acide carboxylique de la formule générale $R^1$—$COOR^2$ et/ou un éther de la formule générale $R^3OR^4$, où $R^1$ et $R^2$ sont tels que définis ci-dessus et $R^3$ et $R^4$, qui peuvent être identiques ou différents, représentent chacun un groupe alcoyle ayant de 1 à 20 atomes de carbone qui peut être substitué par du fluor ou du chlore ou représentent un groupe aryle, alcaryle ou aralcoyle qui peut être substitué par du fluor ou du chlore, sont mis à réagir avec de l'oxyde de carbone et de l'hydrogène à température et pression élevées en

présence d'un système catalytique qui comprend un composé du ruthénium et un composé d'un autre métal du groupe VIII, de l'acide iodhydrique, de l'acide bromhydrique, de l'iode élémentaire et/ou du brome élémentaire étant ajoutés au mélange réactionnel.

2. Un procédé selon la revendication 1, caractérisé en ce que comme matières de départ on utilise des composés des formules générales $R^1$—$COOR^2$ et/ou $R^3OR^4$ où $R^1$, $R^2$, $R^3$ et $R^4$, qui peuvent être identiques ou différents, représentent chacun un groupe alcoyle ayant de 1 à 12 atomes de carbone ou un groupe aryle, alcaryle ou aralcoyle ayant jusqu'à 12 atomes de carbone, tandis que $R^1$ peut aussi représenter un atome d'hydrogène, et de préférence sont identiques et représentent un groupe alcoyle ayant de 1 à 12 atomes de carbone ou un groupe aryle, alcaryle ou aralcoyle ayant jusqu'à 12 atomes de carbone.

3. Un procédé selon la revendication 1 ou 2, caractérisé en ce que comme composé du ruthénium, on utilise du chlorure de ruthénium (III), du trihydrate de chlorure de ruthénium (III), du chlorure de ruthénium (IV), du bromure de ruthénium (III), de l'oxyde de ruthénium ou un sel organique ou complexe de ruthénium.

4. Un procédé selon l'une quelconque des revendications précédentes, caractérisé en ce que comme composé d'un autre métal du groupe VIII on utilise un composé du rhodium comme de l'oxyde de rhodium, de l'hydroxyde de rhodium (III), du chlorure de rhodium (III), du trihydrate de chlorure de rhodium (III), du bromure de rhodium (III), de l'iodure de rhodium (III) ou un sel organique ou complexe de rhodium, de préférence du trihydrate de chlorure de rhodium (III), ou un composé du palladium comme du chlorure de palladium, du dihydrate de chlorure de palladium, du bromure de paladium, de l'iodure de palladium, de l'oxyde de palladium ou un sel organique ou complexe de palladium, de préférence du chlorure de palladium, du dihydrate de chlorure de palladium ou de l'acétate de palladium.

5. Un procédé selon la revendication 4, caractérisé en ce qu'on utilise un composé du rhodium comme composé d'un autre métal du groupe VIII.

6. Un procédé selon l'une quelconque des revendications précédentes, caractérisé en ce qu'on utilise le composé du ruthénium et le composé d'un autre métal du groupe VIII dans un rapport compris entre 50:1 et 1:20, de préférence entre 10:1 et 1:5.

7. Un procédé selon l'une quelconque des revendications précédentes, caractérisé en ce qu'en outre un composé organo-phosphore, organo-arsenic, organo-antimoine, organo-azote, organo-soufre ou organo-oxygène est présent dans le mélange réactionnel comme promoteur.

8. Un procédé selon la revendication 7, dans lequel une amine ou une phosphine est présente dans le mélange réactionnel à raison de moins de 0,5 mole par atome-gramme de ruthénium.

9. Un procédé selon l'une quelconque des revendications précédentes, caractérisé en ce que la réaction est conduite à une température comprise entre 50°C et 200°C et spécialement entre 125°C et 175°C.

10. Un procédé selon l'une quelconque des revendications précédentes, caractérisé en ce que le procédé est mis en oeuvre en utilisant une pression compris entre 20 et 100 bars.